# EUROPÄISCHE PATENTANMELDUNG

(11) **EP 1 529 520 A1**
(43) Veröffentlichungstag der Anmeldung: **11.05.2005**
(21) Anmeldenummer: 04024905.4
(22) Anmeldetag: 20.10.2004
(51) Int. Cl.: A61K 7/48, A61K 31/05, A61K 31/78, A61P 17/00, A61P 17/06

(54) **Verwendung von Licoochalcon A oder eines Licoochalcon A enthaltenden Extraktes aus Radix Glycyrrhizae inflatae gegen postinflammatorische Hyperpigmentierung**

(30) Priorität: 10.11.2003 DE 10352369
(71) Anmelder: Beiersdorf AG, 20253 Hamburg (DE)
(72) Erfinder: Max, Heiner, Dr., 22529 Hamburg (DE); Wolber, Rainer, Dr., 22397 Hamburg (DE); Mummert, Christopher, Dr., 29553 Bienenbüttel (DE); Kolbe, Ludger, Dr., 21255 Dohren (DE); Dieck, Karen tom, 22299 Hamburg (DE); Wensorra, Ursula, 21035 Hamburg (DE)

(57) **Zusammenfassung**

Verwendung von Licochalcon A oder eines Licocalchon A enthaltenden Extraktes aus Radix Glycyrrhizae inflatae in kosmetischen oder dermatologischen Zubereitungen zur Behandlung und Prophylaxe von postinflammatorischen Hautzuständen wie Hyperpigmentierung.

## Beschreibung

Die vorliegende E rfindung betrifft k osmetische b zw. d ermatologische d ermatologische Z ubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Für die Pigmentierung der Haut verantwortlich sind die Melanozyten, welche in der untersten Schicht d er E pidermis, dem S tratum b asale, n eben d en B asalzellen als - je nach Hauttyp entweder vereinzelt oder aber mehr oder weniger gehäuft auftretende - pigmentbildende Zellen vorzufinden sind.

Melanozyten enthalten als charakteristische Zellorganellen Melanosomen, in denen das Melanin gebildet wird. Unter anderem bei Anregung durch UV-Strahlung wird verstärkt Melanin gebildet. Dieses wird über die lebenden Schichten der Epidermis (Keratinozyten) letztlich in die Hornschicht (Corneozyten) transportiert und ruft eine mehr oder weniger ausgeprägte bräunliche bis braun-schwarze Hautfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICA- und DHI-Melanin entstehen über die gemeinsamen Zwischenstufen Dopachinon und Dopachrom. Letzteres wird, teilweise unter Beteiligung weiterer Enzyme, entweder in Indol-5,6-Chinon-Carbonsäure oder in Indol-5,6-Chinon umgesetzt, woraus die beiden genannten Eumelanine entstehen.

Die Entstehung von Phäomelanin läuft unter anderem über die Zwischenprodukte Dopachinon und Cysteinyldopa. Gesteuert wird die Expression der Melanin-synthetisierenden Enzyme durch einen spezifischen Transkriptionsfaktor (microphthalmia- associated transcription factor, MITF). Neben den beschriebenen enzymatischen Prozessen der Melanin-Synthese sind in den Melanosomen noch weitere Proteine für die Melanogenese von Bedeutung. Eine wichtige Rolle scheint hier dem sogenannten p-Protein zuzukommen, wobei die exakte Funktion noch unklar ist.

Neben dem zuvor beschriebenen Prozeß der Melanin-Synthese in den Melanozyten, ist bei der Pigmentierung der Haut auch der Transfer der Melanosomen, deren Verbleib in der Epidermis sowie deren Abbau und der Abbau des Melanins von entscheidender Bedeutung. Es konnte gezeigt werden daß für den Transport der Melanosomen aus den Melanozyten in die Keratinozyten der PAR-2-Rezeptor bedeutsam ist (M. Seiberg et al., 2000, J. Cell. Sci., 113:3093-101).

Ferner haben Größe und Form der Melanosomen Einfluß auf ihre lichtstreuenden Eigenschaften und somit das farbliche Erscheinungsbild der Haut. So findet man bei Schwarzafrikanern verstärkt große spheroidale, einzeln vorliegende Melanosomen, während man bei Kaukasiern eher kleinere, in Gruppen vorkommende Melanosomen vorfindet.

Probleme mit Hyperpigmentierung der Haut haben vielfältige Ursachen bzw. sind Begleiterscheinungen vieler biologischer Vorgänge, z.B. UV-Strahlung (z.B. Sommersprossen, *Ephelides),* genetische Disposition, Fehlpigmentierung der Haut bei der Wundheilung bzw. - vernarbung (postinflammatorische Hyperpigmentierung) oder der Hautalterung (z.B. *Lentigines seniles*).

Nach e ntzündlichen R eaktionen reagiert d as P igmentierungssystem d er H aut m it teilweise entgegengesetzten Reaktionen. Es kann sowohl zu postinflammatorischen Hyper- wie auch Hypopigmentierungen kommen. Postinflammatorische Hypomelanosen treten u. a. häufig in Verbindung mit Atopie, Lupus erythematosus und Psoriasis auf. Die unterschiedlichen Reaktionsformen des Pigmentierungssystems der menschlichen Haut in Folge entzündlicher Erscheinungen sind nur sehr unvollständig verstanden.

Probleme mit postinflammatorischer Hyperpigmentierung treten häufig bei dunkleren Hauttypen auf. Insbesondere bei männlichen Farbigen ist das Problem der *Pseudofollikulitis barbae* bekannt, das mit kosmetisch unerwünschten Fehlpigmentierung einhergeht bzw. diese nach sich zieht. Auch Formen von Melasma, welche insbesondere bei Frauen asiatischer Zugehörigkeit im Gesicht und im Dekolleté - Bereich auftreten, sowie verschiedene Formen der unregelmäßigen Pigmentierung der Haut werden zu den postinflammatorischen Hyperpigmentierungen gezählt. Ferner werden auch dunkle Augenringe als eine Form postinflammatorischen Hyperpigmentierungen angesehen, wobei die zugrunde liegende Entzündung meist subklinisch abläuft.

In vielen Fällen werden derartige postinflammatorische Fehlpigmentierung durch Einwirkung von Sonnenlicht (UV-Licht) noch verstärkt, ohne daß es zu einer UV-induzierten Entzündung (Sonnenbrand) kommt.

Es sind Wirkstoffe und Zubereitungen bekannt, welche der Hautpigmentierung e ntgegenwirken. Im praktischen Gebrauch sind im wesentlichen Präparate auf der Grundlage von Hydrochinon, welche aber einesteils erst nach mehrwöchiger Anwendung ihre Wirkung zeigen, deren übertrieben lange Anwendung andererseits aus toxikologischen Gründen bedenklich ist. Von Albert Kligman et al. wurde eine sogenannte Triformula entwickelt, die eine Kombination aus 0.1% Tretinoin, 5.0% Hydroquinone, 0.1% Dexamethasone darstellt (A. Kligman, 1975, Arch. Dermatol., 111:40-48). Allerdings ist auch diese Formulierung wegen möglicher irreversibler Veränderungen im Pigmentierungssystem der Haut sehr umstritten.

Ferner finden hautschälende Methoden (chemische und mechanische "Peelings") A nwendung, die jedoch häufig entzündliche Reaktionen nach sich ziehen und aufgrund danach eintretender p ostinflammatorischer H yperpigmentierungen sogar zu stärkerer statt verminderter Pigmentierung führen können. All diese gängigen Verfahren, die auch zur Behandlung von postinflammatorischen Hypergigmentierungen angewendet werden, zeichnen sich durch entscheidende Nebenwirkungen aus.

Ziel der nachfolgenden Erfindung war es also, dem nachteiligen Stand der Technik Abhilfe zu verschaffen.

So hat sich insbesondere vor dem Hintergrund der bislang nicht vollständig verstandenen Reaktionen des Pigmentierungssystems der Haut völlig überraschend gezeigt, daß die Verwendung von Licochalcon A oder eines Licocalchon A enthaltenden Extraktes aus Radix Glycyrrhizae inflatae in kosmetischen oder dermatologischen Zubereitungen zur Behandlung und Prophylaxe der postinflammatorischen Hautzuständen hervorragend wirksam ist und so zu einer ebenmäßigeren Pigmentierung der Haut beiträgt.

In einer besonders bevorzugten Verwendungsform gilt dies zur Behandlung von folgenden Hyperpigmentierunsgzuständen: Postinflammatorische Hyperpigmentierung nach entzündliche Reaktionen, insbesondere die mit der Rasur in Verbindung gebracht werden, Melasma, uneven skin tone insbesondere infolge übermäßiger Sonnenbestrahlung. Es zeigte sich, daß in einer besonders bevorzugten Anwendungsform Licochalcone A bzw. Extrakte dieses enthaltend in Kombination mit UV-Filtern eingesetzt werden. Neben der Vorbeugung und Behandlung von postinflammatorischen Hyperpigmentierungen durch die erfindungsgemäßen Zubereitungen als besonders bevorzugte Ausführungsform, zeigten sich ein einer bevorzugten Ausführungsform die erfindungsgemäßen Formulierungen auch bei der Behandlung von Hypogigmentierungen als wirksam.

Als besonders bevorzugte Indikationsgebiete wären postinflammatorische Hyperpigmentierungen infolge der Pseudofollikularis Barbae sowie Melasma zu nennen.

Aufgabe der vorliegenden Erfindung war es somit, Wege zu finden, die die Nachteile des Standes der Technik vermeiden. Insbesondere soll die Wirkung der Behebung der mit der endogenen, chronologischen u nd e xogenen H autalterung verbundenen S chäden u nd d ie Prophylaxe dauerhaft, nachhaltig und ohne das Risiko von Nebenwirkungen sein.

Diesen Übelständen abzuhelfen, war Aufgabe der vorliegenden Erfindung.

Es hat sich überraschenderweise herausgestellt, daß die Verwendung von Licochalcon A oder eines Licocalchon A enthaltenden Extraktes aus Radix Glycyrrhizae inflatae in kosmetischen oder dermatologischen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut den Nachteilen das Standes der Technik abhilft.

Vorteilhaft ist insbesondere eine erfindungsgemäße Verwendung, dadurch gekennzeichnet, daß die Zubereitungen 0,0001 bis 5 Gew.-%, insbesondere 0,001 bis 1 Gew.-%, ganz besonders 0,005 bis 0,15 Gew.-% Licochalcon A enthalten, bezogen auf das Gesamtgewicht der Zubereitung.

Vorteilhaft ist ferner insbesondere e ine e rfindungsgemäße V erwendung, d adurch g ekennzeichnet, daß die Zubereitungen 0,001 bis 10 Gew.-%, insbesondere 0,05 bis 5 Gew.-%, ganz besonders 0,01 bis 2 Gew.-% an einem oder mehreren ethoxylierten oder propoxylierten Rohstoffen enthalten, bezogen auf das Gesamtgewicht der Zubereitung.

Vorteilhaft ist ferner insbesondere e ine e rfindungsgemäße V erwendung, d adurch g ekennzeichnet, daß die Zubereitungen 0,001 bis 10 Gew.-%, insbesondere 0,05 bis 5 Gew.-%, ganz besonders 0,01 bis 2 Gew.-% an einem oder mehreren Polyolen enthalten, bezogen auf das Gesamtgewicht der Zubereitung.

Vorteilhaft ist ferner insbesondere e ine e rfindungsgemäße V erwendung, d adurch g ekennzeichnet, daß die Zubereitungen Licocalchon als Bestandteil von pflanzlichen Extracten, insbesondere von *Radix Glycyrrhizae inflatae*, enthalten.

Die Pflanzenart *Glycyrrhiza inflata* gehört wie das in Europa offizinelle Süßholz *Glycyrrhiza glabra* der Gattung *Glycyrrhiza* an, die zur *Pflanzenfamilie* der *Fabaceae* (Erbsengewächse) gehört. Die Droge *Radix Glycyrrhizae inflatae*, d.h., die Wurzel der Pflanze, ist, beispielsweise in der fernöstlichen Medizin, gebräuchlich. Die Verwendung der Droge als Entzündungshemmer ist ebenfalls bekannt.

Ein Bestandteil des wäßrigen Auszugs aus Radix Glycyrrhizae inflatae ist das Licochalcon A, welches sich durch die folgende Strukturformel auszeichnet:

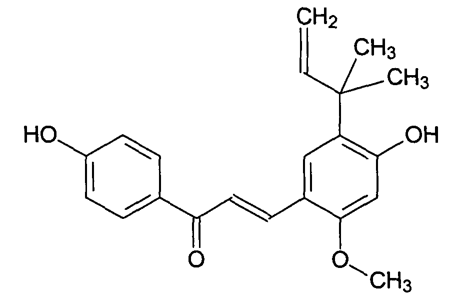

Es wird angenommen, daß diese Substanz, möglicherweise in Synergie mit den übrigen Bestandteilen des Extraktes, Anteil an der erfindungsgemäßen Wirkung besitzt.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.-% an einem wäßrigen Extrakt aus *Radix Glycyrrhizae inflatae* enthalten, bezogen auf das Gesamtgewicht der Zubereitung.

Es ist erfindungsgemäß vorteilhaft, wenn die kosmetischen oder dermatologischen Zubereitungen 0,001 bis 10 Gew.-%, insbesondere 0,05 bis 5 Gew.-%, ganz besonders 0,01 bis 2 Gew.-% an einem oder mehreren Polyolen enthalten, bezogen auf das Gesamtgewicht der Zubereitung.

Insbesondere ist vorteilhaft, als Polyol das Butylenglycol zu wählen.

Ganz besonders vorteilhaft ist es, von einem Extrakt auszugehen, der unter der Bezeichnung Polyol Soluble Licorice Extract P-U von der Firma Maruzen vertrieben wird.

Ferner ist es vorteilhaft, Licochalcone A in anderen Vehikelsystemen in einer Konzentration von 0,0001 bis 5 Gew.-%, insbesondere 0,001 bis 1 Gew.-%, ganz besonders 0,005 - 0,05 Gew.-% zu verwenden.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), s owie K oniferylbenzoat d es Benzoëharzes, R utinsäure u nd d eren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist a uch m öglich u nd vorteilhaft im S inne d er vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in d er R egel e in oder m ehrere M edikamente in w irksamer K onzentration. D er E infachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind gegebenenfalls auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu e rstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können e rfindungsgemäße Zubereitungen Substanzen enthalten, die UV-Strahlung im U VB-Bereich a bsorbieren, w obei d ie G esamtmenge d er F iltersubstanzen z.B. 0,1 Gew.-% b is 3 0 G ew.-%, vorzugsweise 0,5 b is 10 Gew.-%, i nsbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder d ermatologischen Zubereitungen e nthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase d er E mulsionen, Oleogele b zw. H ydrodispersionen oder L ipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele O/W-Crèmes

| **Beispiel Nr. 1** | |
|---|---|
| Glycerylsterat selbstemulgierend | 4,00 |
| PEG-40-Stearat | 1,00 |
| Cetylalkohol | 3,00 |
| Caprylic-/Capric-Triglycerid | 5,00 |
| Paraffinum liquidum | 5,00 |
| Licochalcon A | 0,05 |
| Tocopherol | 0,1 |
| Na₃HEDTA | 0,1 |
| Konservierungsmittel, Parfum | q.s. |
| Polyacrylsäure | 3,00 |
| Natronlauge 45% | q.s |
| Glycerin | 5,00 |
| Wasser | ad100 |

| **Beispiel Nr. 2** | |
|---|---|
| Glycerylsterat selbstemulgierend | 3,00 |
| Stearinsäure | 1,00 |
| Cetylalkohol | 2,00 |
| Caprylic-/Capric-Triglycerid | 3,00 |
| Dicaprylylether | 4,00 |
| Paraffinum liquidum | 2,00 |
| Licochalcon A | 0,01 |
| Konservierungsmittel, Parfum | q.s. |
| Polyacrylsäure | 0,1 |
| Natronlauge 45% | q.s. |
| Glycerin | 3,00 |
| Butylenglycol | 3,00 |
| Wasser | ad100 |

| **Beispiel Nr. 3** | |
|---|---|
| Glycerylstearatcitrat | 2,00 |
| Stearylalkohol | 2,00 |
| Lanolinalkohol | 1,00 |
| Caprylic-/Capric-Triglycerid | 4,00 |
| Paraffinum liquidum | 8,00 |
| Dimethicon | 1,00 |
| Licochalcon A | 0,04 |
| Konservierungsmittel, Parfum | q.s. |
| Natronlauge 45% | q.s. |
| Glycerin | 7,50 |
| Wasser | ad100 |

| **Beispiel Nr. 4** | |
|---|---|
| Glycerylstearatcitrat | 2,00 |
| Stearylalkohol | 2,00 |
| Lanolinalkohol | 1,00 |
| Caprylic-/Capric-Triglycerid | 4,00 |
| Paraffinum liquidum | 8,00 |
| Dimethicon | 1,00 |
| Licochalcon A | 0,03 |
| Konservierungsmittel, Parfum | q.s. |
| Natronlauge 45% | q.s. |
| Glycerin | 7,50 |
| Dihydroxyaceton | 1,00 |
| Wasser | ad100 |

| **Beispiel Nr. 5** | |
|---|---|
| Polyglyceryl-3-Methylglucose-Distearat | 3,00 |
| Cetylalkohol | 3,00 |
| Caprylic-/Capric-Triglycerid | 3,00 |
| Dicaprylylether | 2,00 |
| Paraffinum liquidum | 3,00 |
| Licochalcon A | 0,25 |
| Na3HEDTA | 0,1 |
| Konservierungsmittel, Parfum | q.s. |
| Polyacrylsäure | 0,1 |
| Natronlauge 45% | q.s. |
| Glycerin | 3,00 |
| Wasser | ad100 |

| **Beispiel Nr. 6** | |
|---|---|
| Glycerylstearatcitrat | 2,00 |
| Sorbitanstearat | 2,00 |
| Cetylstearylalkohol | 2,00 |
| Caprylic-/Capric-Triglycerid | 3,00 |
| Octyldodecanol | 2,00 |
| Dicaprylylether | 1,00 |
| Licochalcon A | 0,0125 |
| Tocopherol | 0,20 |
| Konservierungsmittel, Parfum | q.s. |
| Polyacrylsäure | 0,1 |
| Natronlauge 45% | q.s. |
| Glycerin | 3,00 |
| Wasser | ad100 |

### Beispiele O/W-Crèmes

| **Beispiel Nr. 7** | |
|---|---|
| Glycerylsterat selbstemulgierend | 5,00 |
| Stearylalkohol | 2,00 |
| Caprylic-/Capric-Triglycerid | 2,00 |
| Octyldodecanol | 2,00 |
| Dimethicon Polydimethylsiloxan | 2,00 |
| Titandioxid | 2,00 |
| 4-Methylbenzylidencampher | 1,00 |
| Butylmethoxy-dibenzoylmethan | 0,50 |
| Licochalcon A | 0,02 |
| Konservierungsmittel, Parfum | q.s. |
| Polyacrylsäure | 0,15 |
| Natronlauge 45% | q.s. |
| Glycerin | 3,00 |
| Wasser | ad 100 |

| **Beispiel Nr. 8** | |
|---|---|
| Glycerylstearatcitrat | 2,00 |
| Cetylstearylalkohol | 3,00 |
| C12-15 Alkylbenzoat | 2,00 |
| Octyldodecanol | 2,00 |
| Paraffinum liquidum | 4,00 |
| Licochalcon A | 0,125 |
| 2,4-Bis-(4-(2-ethyl-hexyloxy-)2-hydroxyl)-phenyl)-6-(4-methoxyphenyl)-(1,3,5)-triazin | 1,0 |
| Dihydroxyaceton | 0,5 |
| Konservierungsmittel, Parfum | q.s. |
| Polyacrylsäure | 0,1 |
| Natronlauge 45% | q.s. |
| Butylenglycol | 3,00 |
| Ethanol | 3,00 |
| Wasser | ad 100 |

| **Beispiel Nr. 9** | |
|---|---|
| Glycerylstearatcitrat | 2,00 |
| Cetylstearylalkohol | 1,00 |
| C12-15 Alkylbenzoat | 3,00 |
| Paraffinum liquidum | 2,00 |
| Licochalcon A | 0,05 |
| 2,4-Bis-(4-(2-ethyl-hexyloxy-)2-hydroxyl)-phenyl)-6-(4-methoxyphenyl)-(1,3,5)-triazin | 3,0 |
| Ethylendiamintetraessigsäure Trinatrium | 0,20 |
| Konservierungsmittel, Parfum | q.s. |
| Xanthan Gummi | 0,20 |
| Natronlauge 45% | q.s. |
| Glycerin | 3,00 |
| Wasser | ad 100 |

| **Beispiel Nr. 10** | |
|---|---|
| Stearinsäure | 2,50 |
| Cetylalkohol | 3,00 |
| Octyldodecanol | 4,00 |
| Cyclisches Dimethylpolysiloxan | 0,50 |
| Licochalcon A | 0,2 |
| Konservierungsmittel, Parfum | q.s. |
| Polyacrylsäure | 0,05 |
| Natronlauge 45% | q.s. |
| Glycerin | 5,00 |
| Ethanol | 3,00 |
| Wasser | ad 100 |

| **Beispiel Nr. 11** | |
|---|---|
| Stearinsäure | 3,50 |
| Cetylalkohol | 4,50 |
| Cetylstearylalkohol | 0,50 |
| Octyldodecanol | 6,00 |
| Cyclisches Dimethylpolysiloxan | 2,00 |
| 4-Methylbenzylidencampher | 1,00 |
| Butylmethoxy-dibenzoylmethan | 0,50 |
| Licochalcon A | 0,10 |
| 2,4-Bis-(4-(2-ethyl-hexyloxy-)2-hydroxyl)-phenyl)-6-(4- | 0,5 |
| methoxyphenyl)-(1,3,5)-triazin | |
| Dihydroxyaceton | 0,5 |
| Tocopherol | 0,05 |
| Ethylendiamintetraessigsäure Trinatrium | 0,20 |
| Konservierungsmittel, Parfum | q.s. |
| Polyacrylsäure | 0,05 |
| Natronlauge 45% | q.s. |
| Glycerin | 3,00 |

### Beispiele W/O-Emulsionen

| **Beispiel Nr. 12** | |
|---|---|
| Polyglyceryl-2-dipolyhydroxystearat | 5,00 |
| 2,4-Bis-(4-(2-ethyl-hexyloxy-)2-hydroxyl)-phenyl)-6-(4-methoxyphenyl)-(1,3,5)-triazin | 2,00 |
| Diethylhexyl Butamidotriazon | 3,00 |
| Octocrylen | 7,00 |
| Diethylhexyl Butamidotriazon | 1,00 |
| Phenylen-1,4-bis-(mononatrium,-2-benzimidazyl-5,7-disulfonsäure) | 1,00 |
| Phenylbenzimidazol Sulfonsäure | 0,50 |
| Zinkoxid | 3,00 |
| Dicaprylylether | 10,00 |
| Dicaprylylcarbonat | 5,00 |
| Phenylmethylpolysiloxan | 2,00 |
| PVP Hexadecencopolymer | 0,50 |
| Glycerin | 3,00 |
| Magnesiumsulfat | 1,00 |
| Tocopherolacetat | 0,50 |
| Licochalcon A | 0,05 |
| Konservierungsmittel, Parfum | q.s. |
| Ethanol | 3,00 |
| Wasser | ad 100 |

| **Beispiel Nr. 13** | |
|---|---|
| Cetyldimethiconcopolyol | 2,50 |
| 2-Ethylhexyl Methoxyzinnamat | 8,00 |
| 2,4-Bis-(4-(2-ethyl-hexyloxy-)2-hydroxyl)-phenyl)-6-(4-methoxyphenyl)-(1,3,5)-triazin | 2,50 |
| Diethylhexyl Butamidotriazon | 1,00 |
| 4-Methylbenzylidencampher | 2,00 |
| Octocrylen | 2,50 |
| Phenylen-1,4-bis-(mononatrium,-2-benzimidazyl-5,7-disulfonsäure) | 2,00 |
| Titandioxid | 2,00 |
| Zinkoxid | 1,00 |
| Dimethicon Polydimethylsiloxan | 4,00 |
| Phenylmethylpolysiloxan | 25,00 |
| Octoxyglycerin | 0,30 |
| Glycerin | 7,50 |
| Glycinsoja | 1,00 |
| Magnesiumsulfat | 0,50 |
| Licochalcon A | 0,02 |
| Konservierungsmittel, Parfum | q.s. |
| Wasser | ad 100 |

| **Beispiel Nr. 14** | |
|---|---|
| PEG-30-dipolyhydroxystearat | 5,00 |
| Butylmethoxy-dibenzoylmethan | 2,00 |
| Ethylhexyl Triazon | 3,00 |
| Octocrylen | 4,00 |
| Phenylen-1,4-bis-(mononatrium,-2-benzimidazyl-5,7-disulfonsäure) | 0,50 |
| Titandioxid | 1,50 |
| Zinkoxid | 2,00 |
| Paraffinum liquidum | 10,0 |
| Butylen-Glycol-Dicaprylat/-Dicaprat | 2,00 |
| Dicaprylylcarbonat | 6,00 |
| Dimethicon Polydimethylsiloxan | 1,00 |
| Shea Butter | 3,00 |
| Octoxyglycerin | 1,00 |
| Glycinsoja | 1,50 |
| Magnesiumchlorid | 1,00 |
| Tocopherolacetat | 0,25 |
| Licochalcon A | 0,125 |
| Konservierungsmittel, Parfum | q.s. |
| Ethanol | 1,50 |
| Wasser | ad 100 |

| **Beispiel Nr. 15** | |
|---|---|
| Cetyldimethiconcopolyol | 4,00 |
| 2-Ethylhexyl Methoxyzinnamat | 5,00 |
| 2,4-Bis-(4-(2-ethyl-hexyloxy-)2-hydroxyl)- | 2,00 |
| phenyl)-6-(4-methoxyphenyl)-(1,3,5)-triazin | |
| Butylmethoxy-dibenzoylmethan | 1,00 |
| Ethylhexyl Triazon | 4,00 |
| 4-Methylbenzylidencampher | 4,00 |
| Diethylhexyl Butamidotriazon | 2,00 |
| Phenylbenzimidazol Sulfonsäure | 3,00 |
| Zinkoxid | 0,50 |
| C₁₂₋₁₅ Alkyl-Benzoat | 9,00 |
| Butylen-Glycol-Dicaprylat/-Dicaprat | 8,00 |
| Dimethicon Polydimethylsiloxan | 5,00 |
| PVP Hexadecencopolymer | 0,50 |
| Glycerin | 7,50 |
| Magnesiumsulfat | 0,50 |
| Licochalcon A | 0,20 |
| Konservierungsmittel, Parfum | q.s. |
| Wasser | ad 100 |

| **Beispiel Nr. 16** | |
|---|---|
| Polyglyceryl-2-dipolyhydroxystearat | 4,50 |
| 2-Ethylhexyl Methoxyzinnamat | 4,00 |
| 2,4-Bis-(4-(2-ethyl-hexyloxy-)2-hydroxyl)- | 2,50 |
| phenyl)-6-(4-methoxyphenyl)-(1,3,5)-triazin | |
| Diethylhexyl Butamidotriazon | 3,00 |
| Ethylhexyl Triazon | |
| 4-Methylbenzylidencampher | 2,00 |
| Octocrylen | 2,50 |
| Phenylbenzimidazol Sulfonsäure | 2,00 |
| Titandioxid | 3,00 |
| Paraffinum liquidum | 8,00 |
| Dicaprylylether | 7,00 |
| Butylen-Glycol-Dicaprylat/-Dicaprat | 4,00 |
| Phenylmethylpolysiloxan | 2,00 |
| PVP Hexadecencopolymer | 1,00 |
| Octoxyglycerin | 0,50 |
| Glycerin | 2,50 |
| Magnesiumchlorid | 0,70 |
| Tocopherolacetat | 1,00 |
| Licochalcon A | 0,25 |
| Konservierungsmittel, Parfum | q.s. |
| Ethanol | 1,00 |
| Wasser | ad 100 |

### Beispiele W/O Emulsionen

| **Beispiel Nr.** | **17** | **18** |
|---|---|---|
| Polyglyceryl-2-dipolyhydroxystearat | 4,00 | 5,00 |
| Lanolinalkohol | 0,50 | 1,50 |
| Isohexadecan | 1,00 | 2,00 |
| Myristyl-Myristat | 0,50 | 1,50 |
| Vaseline | 1,00 | 2,00 |
| Butylmethoxy-dibenzoylmethan | 0,50 | 1,50 |
| 4-Methylbenzylidencampher | 1,00 | 3,00 |
| Butylen-Glycol-Dicaprylat/-Dicaprat | 4,00 | 5,00 |
| Shea Butter | - | 0,50 |
| Butylenglycol | - | 6,00 |
| Octoxyglycerin | - | 3,00 |
| Glycerin | 5,00 | - |
| Tocopherolacetat | 0,50 | 1,00 |
| Licochalcon A | 0,2 | 0,1 |
| EDTA | 0,20 | 0,20 |
| Konservierungsmittel | q.s. | q.s. |
| Ethanol | - | 3,00 |
| Parfum | q.s. | q.s. |
| Wasser | ad 100 | ad 100 |

### Beispiel (W/O-Creme)

| **Beispiel Nr. 19** | |
|---|---|
| Polyglyceryl-3-Diisostearat | 3,50 |
| Glycerin | 3,00 |
| Polyglyceryl-2-Dipolyhydroxystearat | 3,50 |
| Licochalcon A | 0,1 |
| Konservierungsmittel | q.s. |
| Parfum | q.s. |
| Magnesiumsulfat | 0,6 |
| Isopropylstearat | 2,0 |
| Caprylylether | 8,0 |
| Cetearylisononanoat | 6,0 |
| Wasser | ad 100 |

### Beispiel (W/O-Emulsion):

| **Beispiel Nr. 20** | |
|---|---|
| Triceteareth-4-Phosphat | 0,80 |
| Butylhydroxytoluol | 0,05 |
| Glyceryllanolat | 1,70 |
| Cyclomethicon | 2,20 |
| Isopropylpalmitat | 1,00 |
| Licochalcon A | 0,10 |
| Polyacrylsäure | 0,50 |
| Ethylendiamintetraessigsäure | 1,00 |
| Natriumhydroxid | q.s. |
| Zitronensäure | 0,01 |
| Konservierungsmittel | q.s. |
| Parfum | q.s. |
| Wasser | ad 100 |

## Patentansprüche

1. Verwendung von Licochalcon A o der e ines L icocalchon A e nthaltenden E xtraktes a us Radix Glycyrrhizae inflatae in kosmetischen oder dermatologischen Zubereitungen zur Behandlung und Prophylaxe der postinflammatorischen Hautzuständen.

2. Verwendung nach Anspruch 1, **dadurch gekennzeichnet, daß** die Zubereitungen 0,0001 bis 5 Gew.-%, insbesondere 0,001 bis 1 Gew.-%, ganz besonders 0,005 bis 0,15 Gew.-% Licochalcon A enthalten, bezogen auf das Gesamtgewicht der Zubereitung.

3. Verwendung nach Anspruch 1, **dadurch gekennzeichnet, daß** die Zubereitungen 0,001 bis 10 Gew.-%, insbesondere 0,05 bis 5 Gew.-%, ganz besonders 0,01 bis 2 Gew.-% an einem oder mehreren Polyolen enthalten, bezogen auf das Gesamtgewicht der Zubereitung.
